# EUROPÄISCHE PATENTSCHRIFT

(11) **EP 2 766 835 B1**
(45) Veröffentlichungstag und Bekanntmachung des Hinweises auf die Patenterteilung: **17.04.2019**
(21) Anmeldenummer: 12798589.3
(22) Anmeldetag: 12.10.2012
(51) Int. Cl.: G06F 19/00

(54) **PROBENENTNAHMEVERFAHREN ZUR VERWECHSLUNGSSICHEREN DURCHFÜHRUNG EINER PROBENENTNAHME**
SAMPLING METHOD FOR PERFORMING SAMPLING IN AN UNMISTAKEABLE MANNER
PROCÉDÉ D'ÉCHANTILLONNAGE DESTINÉ À LA MISE EN OEUVRE SANS RISQUE DE CONFUSION D'UN PROCÉDÉ D'ÉCHANTILLONNAGE

(30) Priorität: 12.10.2011 AT 14772011
(43) Veröffentlichungstag der Anmeldung: 20.08.2014
(73) Patentinhaber: Greiner Bio-One GmbH, 4550 Kremsmünster (AT)
(72) Erfinder: RAMPETSREITER, Christoph, A-4550 Kremsmünster (AT)
(74) Vertreter: Burger, Hannes
(86) Internationale Anmeldenummer: PCT/AT2012/050162
(87) Internationale Veröffentlichungsnummer: WO 2013/052990

(56) Entgegenhaltungen:
- WO-A2-01/20532
- WO-A2-03/021525
- WO-A2-2005/111086
- WO-A2-2008/109666
- AT-B- 413 791

## Beschreibung

Die Erfindung betrifft ein Probenentnahmeverfahren zur verwechslungssicheren Durchführung einer Probenentnahme ausgeführt auf einem Datenverarbeitungssystem mit einem Datenspeicher.

Bei einer Entnahme einer Probe ist es bekannt, eine Zuordnung der entnommenen Probe zur Probenquelle sicher und nachvollziehbar zu dokumentieren. Insbesondere bei der hier bevorzugten Anwendung im Gesundheitsbereich, handelt es sich bei den Proben zumeist um biologische Proben einer Person, beispielsweise eines Patienten in einem Krankenhaus bzw. in einer Arztpraxis. Diese Zuordnung wird beispielsweise dadurch erreicht, dass für einen Patienten ein spezifisches Kennzeichnungsetikett generiert wird, welches nach der Probenentnahme am Probenbehälter angebracht wird und somit die Zuordnung über diese angebrachte Kennung hergestellt wird. Im täglichen Laborbetrieb, insbesondere bei einem großen Durchsatz an Patienten, ist diese Art der Zuordnung jedoch sehr fehleranfällig. Das Markierungskennzeichen, zumeist ein Klebeetikett, wird von einem Bediener vor oder nach der Probenentnahme am Probenbehälter angebracht, wobei es ausschließlich der Sorgfalt des Abnehmers obliegt, dass es hier zu keiner Verwechslung der Probenbehälter eines Patienten kommt. Auch besteht beim Einsatz eines derartigen Verfahrens stets die Gefahr, dass Probenbehälter verschiedener Patienten vertauscht werden und somit falsche biologische Probenwerte ermittelt werden. Insbesondere kann bei Probenabnahmen ggf. auch eine bestimmte Entnahmereihenfolge vorgeschrieben sein, beispielsweise dann, wenn durch eine Probenentnahme die Probenquelle verunreinigt wird. Dies kann geschehen, wenn vor der Probenentnahme dem Patienten ein Mittel beigebracht wird, um so eine Reaktion des Körpers auf das Mittel zu detektieren. Alle dieser Mittelgabe nachgelagerten Probenentnahmen sind jedoch durch das gegebene Mittel verunreinigt.

Da Analysegeräte zur Auswertung der biologischen Proben zumeist vollautomatisch arbeiten und insbesondere das aufgebrachte Kennzeichnungsmerkmal automatisch auslesen müssen, wird im täglichen Einsatz immer mehr von der manuellen Aufbringung eines Kennzeichnungsmerkmals abgekommen. Gerade bei Klebeetiketten besteht stets die Gefahr, dass diese unsachgemäß bzw. falsch ausgerichtet ausgebracht werden, sodass vom nachgelagerten Laboranalysegerät, welches zumeist sehr enge Toleranzbereiche für die Aufbringung des Kennzeichnungsmerkmals vorgibt, dieses nicht ausgelesen und somit die entnommene Probe gegebenenfalls ungültig wird. Dies bedeutet jedoch stets, dass sich der Patient der Probenentnahme erneut unterziehen muss. Daher werden immer öfter Probeentnahmebehälter eingesetzt, auf welchen bereits eine eindeutige Identifikationskennung aufgebracht ist. Somit lässt sich zwar die Problematik der Aufbringung von Kennzeichnungsmerkmalen verhindern, eine eindeutige Zuordnung zu einem Patienten bzw. eine Sicherstellung einer korrekten und gültigen Probenentnahme ist damit jedoch noch nicht gewährleistet. Eine eindeutige Nachvollziehbarkeit der Probenentnahme und insbesondere eine korrekte Durchführung bzw. Reihenfolge der Probenentnahme ist mit dem bekannten Verfahren nicht gewährleistet. Insbesondere ist nicht gewährleistet, dass eine systematisch korrekte Probenentnahme erfolgt, sodass eine gegebenenfalls fehlerhafte Probenentnahme nicht erst bei einer nachgelagerten Analyse erkannt wird. Beispielsweise wird nicht geprüft bzw. kontrolliert, ob für eine spezifische Probenentnahme der dafür vorgesehene Probenentnahmebehälter verwendet wird.

Die Druckschrift WO 2005/111086 A2 offenbart ein Probeentnahmeverfahren, welches auf der Erstellung und Verfolgung von Etiketten beruht.

Die Aufgabe der Erfindung liegt also darin ein Probenentnahmeverfahren zu schaffen, welches eine eindeutige Nachvollziehbarkeit gewährleistet und welches zusätzlich systematische Fehler bei der Probenentnahme verhindert.

Die Aufgabe der Erfindung wird dadurch gelöst, dass das Probenentnahmeverfahren auf einem Datenverarbeitungssystem mit einem Datenspeicher ausgeführt wird, wobei im Datenspeicher eine Mehrzahl probenbezogener und analysebezogener Datensätze hinterlegt sind und wobei die analysebezogenen Datensätze relational mit den probenbezogenen Datensätzen verknüpft sind. Ferner ist eine Benutzerschnittstelle mit einer Ein- Ausgabevorrichtung vorhanden, welche Benutzerschnittstelle mit dem Datenverarbeitungssystem kommunikativ verbunden ist. Dabei wird ein erstes Kennzeichnungsmerkmal einer ersten Person erfasst und aus dem Datenspeicher die analysebezogenen Datensätze ausgelesen und eine Analysekennung an einem Anzeigemittel der Ein- Ausgabevorrichtung dargestellt. Zur Vorbereitung von Behandlungen bzw. zur Diagnose besteht im diagnostischen Prozess - bzw. im Laboralltag eine wesentliche Tätigkeit darin, für einen Patienten eine Analyse relevanter biologischer Kennwerte zu bestimmen, bspw. ein Blutbild. Dabei ist es jedoch wichtig, dass die zu bestimmenden Kennwerte eindeutig dem Patienten zugeordnet sind. Da einem Patienten bzw. allgemein einer ersten Person ein erstes Kennzeichnungsmerkmal zugeordnet ist, wird eine eindeutige Identifikation der Person im Krankenhaus gewährleistet. In den analysebezogenen Datensätzen sind alle möglichen durchführbaren Analysen hinterlegt, wobei am Anzeigemittel als Analysekennung bspw. eine alphanumerische Kennung der entsprechenden Analyse dargestellt wird. Unter einer alphanumerischen Kennung werden bspw. jene Kennungen verstanden, die im Krankenhaus- bzw. Laboralltag gebräuchlich sind, um spezifische Kennwerte zu kennzeichnen, welche aus einer Probe ermittelt werden können. Beispiele dafür sind LDH, GGT, CRP, PTT, wobei diese Aufzählung keinesfalls abschließend zu sehen ist, da dem Fachmann noch weitere derartige alphanumerische Kennungen bekannt sind.

Nach Festlegen einer Teilmenge von Analysen durch Auswählen zumindest einer Analysenkennung mittels der Eingabevorrichtung, wird aus dem ersten Kennzeichnungsmerkmal und der Teilmenge ein Auftragsdatensatz gebildet und dieser im Datenspeicher hinterlegt. Die mit der Teilmenge relational verknüpften probenbezogenen Datensätze werden ebenfalls ausgelesen und ein im probenbezogenen Datensatz hinterlegten Proben-Kennzeichnungsmerkmals, insbesondere ein Abbild, wird am Anzeigemittel dargestellt.

Danach wird von der zweiten Person die Probenentnahme durchgeführt und ein Kennzeichnungsmerkmal eines Probenbehälters erfasst und daraus eine eindeutige Kennung ausgelesen und diese Kennung im Auftragsdatensatz hinterlegt. Ebenso wird ein zweites Kennzeichnungsmerkmal einer zweiten Person erfasst und auch im Auftragsdatensatz hinterlegt. Die zweite Person ist üblicher Weise der Probenabnehmer und muss als solcher eine entsprechende Qualifikation aufweisen, die Probe ent- bzw. abnehmen zu dürfen. Im Sinne einer Qualitätssicherung ist somit klar nachvollziehbar, welche zweite Person die Probenabnahme durchgeführt hat. Eine zweite Person mit zu geringer Qualifikation kann somit bereits beim Erfassen des zweiten Kennzeichnungsmerkmals abgewiesen werden.

Um die biologische Probe zuverlässig auswerten zu können ist ferner sicher zu stellen, dass der korrekte Probenbehälter für die Abnahme verwendet wird. Daher wird eine Typinformation aus dem eindeutigen Kennungsmerkmal des Probenbehälters ausgelesen und mit einer im analysebezogenen Datensatz der Teilmenge hinterlegten Typinformation verglichen. Wurde ein falscher Probenbehälter verwendet liefert der Vergleich einen Fehler, so dass sofort darauf reagiert werden kann und die Probe ggf. erneut abgenommen werden kann. Insbesondere erhält die zweite Person am Anzeigemittel einen eindeutigen Hinweis, dass für die durch die Teilmenge festgelegte Analyse, ein falscher Probenbehälter verwendet wurde.

Unter dem eindeutigen Kennungsmerkmal wird ein Identifikationsmerkmal des Probenbehälters verstanden, welches diesem im Wesentlichen nicht manipulierbar zugeordnet ist, bspw. kann dies bereits bei der Produktion des Behälters erfolgen. Das Kennungsmerkmal kann nun als Container für behälterspezifische Information verstanden werden, gemäß der gegenständlichen Ausbildung ist u.a. die Typinformation des Probenbehälters hinterlegt.

Es ist jedoch auch möglich, dass nicht nur die jeweiligen Kennungen der einzelnen Analysen dargestellt werden, sondern es sind auch Gruppierungen möglich, so dass anstelle der einzelnen zu ermittelnden biologischen Kennwerte ein Oberbegriff dargestellt wird. So könnte bspw. eine Kennung "großes Blutbild" dargestellt werden, unter der Kennung alle relevanten zu erfassenden einzelnen Kennwerte zusammengefasst sind. Dazu kann in den analysebezogenen Datensätzen bspw. eine Gruppenkennung hinterlegt sein, oder es ist im Datenspeicher zusätzlich eine Gruppenzuordnungstabelle hinterlegt, welche eine relationale Verknüpfung zwischen den analysebezogenen Datensätzen festlegt.

In der Beschreibung wird zumeist auf die Abläufe in einem Krankenhaus Bezug genommen. Es wird jedoch ausdrücklich festgehalten, dass das Verfahren nicht auf den Einsatz in Krankenhäusern beschränkt ist, sondern überall dort einsetzbar ist, wo biologische Proben einer Person genommen werden müssen. Auch sind im Text die Begriffe erste Person und Patient bzw. zweiter Person und Abnehmer gleichbedeutend.

Als Datenspeicher wird hierin allgemein ein Repository verstanden, in welchem eine Mehrzahl von Datensätzen abgelegt werden kann. Insbesondere wird darunter eine Datenbank mit einem DBMS (Datenbankmanagementsystem) verstanden. Es ist jedoch auch möglich, dass der Datenspeicher durch eine Datendatei gebildet ist, in welcher Datei die Datensätze abgelegt werden. Im Hinblick auf sog. Cloud-Systeme, kann der Datenspeicher auch durch einen virtuell aus der Cloud in das Datenverarbeitungssystem eingebundenen Datenspeicher gebildet sein.

Die Anforderungsanweisung wird dabei von einem Prozess-Verwaltungssystem an das Datenverarbeitungssystem übermittelt, wobei das Prozess-Verwaltungssystem und das Datenverarbeitungssystem über ein Datenaustauschprotokoll, insbesondere HL7, kommunizieren. Da die Abnahme und Analyse biologischer Proben gelegentlich ein eigenständiges System bzw. eine eigenständige Abteilung ist, auch innerhalb eines Krankenhauses, hat diese Weiterbildung den Vorteil, dass von einem zentralen Verwaltungssystem, wo zumeist auch die Patientendaten verwaltet werden, eine Anforderung für eine Probenentnahme generiert werden kann und somit direkt vom gegenständlichen Probenentnahmeverfahren übernommen werden kann.

Da eine optische Darstellung eines Artikels stets eine geringere Verwechslungsgefahr gegenüber einer alphanumerischen Kennzeichnung birgt, wird als Proben-Kennzeichnungsmerkmal eine Abbildung eines Containers aus dem probenbezogenen Datensatz ausgelesen und dargestellt. Da es sich als vorteilhaft erwiesen hat, die Probenbehälter durch farblich unterschiedliche Verschlusskappen zu kennzeichnen, wird die anspruchsgemäß dargestellte Abbildung bevorzugt eine farbige Darstellung der Verschlusskappe umfassen. Die Verschlusskappe kann ferner einen zusätzlichen farblichen Codierungsring aufweisen, zumeist ist diese stirnseitig angeordnet, so dass bevorzugt eine Schrägansicht der Verschlusskappe dargestellt wird. Es ist ferner vorgesehen, dass aus der eindeutigen Kennung des Probenbehälters zumindest eines der Gruppe Herstellerkennung, Produktionszeitstempel, Verwendungszeitraum, Chargennummer und Artikelnummer ausgelesen wird. Ein Vorteil liegt bspw. darin, dass somit eine Nachverfolgung des einzelnen Behälters bis hin zur Herstellung möglich ist. Ein Probenbehälter ist Teil einer Herstellungscharge wobei es aufgrund der Chargengröße zumeist vorkommen wird, dass die Behälter einer Charge auf eine Vielzahl von Anwender verteilt ausgeliefert werden. Wird nun bei einem fehlerhaften Probenbehälter bspw. die Herstellerkennung an einen Erfassungsdienst des Herstellers übermittelt, kann darüber ein Herstellungsproblem einer Charge sehr rasch erkannt werden. Die Übermittlung erfolgt bevorzugt automatisiert, in dem für einen fehlerhaften Probenbehälter das Kennzeichnungsmerkmal erfasst und vom Datenverarbeitungssystem die Kennung automatisch an den Erfassungsdienst übermittelt wird. Somit kann vom Erfassungsdienst aus den eingehenden Kennungen fehlerhafter Probenbehälter eine Qualitätsinformation für die gesamte Charge abgeleitet werden.

Gemäß einer Weiterbildung wird das erste und/oder zweite Kennzeichnungsmerkmal kontaktlos ausgelesen was bspw. den Vorteil hat, dass eine Verwechslung der Kennung der ersten Person durch eine fehlerhafte Eingabe dieser Kennung vermieden wird. Das erste Kennzeichnungsmerkmal kann fest verbunden am Patienten angebracht sein bspw. als Armband, so dass ein Vorweisen ggf. umständlich durchgeführt werden müsste, oder es lässt der Gesundheitszustand des Patienten ein Vorweisen des ersten Kennzeichnungsmerkmals nicht zu, so dass ein kontaktloses Auslesen die Erfassungssicherheit deutlich erhöht. Das zweite Kennzeichnungsmerkmal ist dem Probenabnehmer zugeordnet bzw. wird von diesem getragen und muss von diesem, bei der Durchführung der Probenentnahme bzw. bei Handlungen im Bezug zum Auftragsdatensatz, erfasst werden. Da dies im Tagesverlauf recht oft geschehen wird, hat die Weiterbildung u.a. die Vorteile, dass einerseits eine Zeitersparnis erreicht wird und andererseits eine Fehleingabe durch eine Unachtsamkeit aufgrund der sich stets wiederholenden Erfassung des zweiten Kennzeichnungsmerkmals verhindert wird. Falls das kontaktlose Auslesen des ersten und/oder zweiten Kennzeichnungsmerkmals fehlschlägt ist vorgesehen, dass das Kennzeichnungsmerkmal auch über ein Eingabemittel, bspw. eine Tastatur, erfasst werden kann.

Eine Weiterbildung nach der als erstes und/oder zweites Kennzeichnungsmerkmal ein Strichcode einer Kennungsmarke ausgelesen wird hat den Vorteil, dass optische Strichcode-Lesegeräte sehr weit verbreitet sind und damit oftmals keine zusätzliche Gerätschaft für das gegenständliche Probenentnahmeverfahren erforderlich ist. Auch sind Einwegarmbänder mit einem Strichcode zur eindeutigen Zutritts- bzw. Personenkennzeichnung ebenfalls weit verbreitet und zumeist bereits im täglichen Einsatz im Gesundheitswesen, insbesondere in Krankenhäusern.

Nach einer Weiterbildung wird als erstes und/oder zweites Kennzeichnungsmerkmal eine Kennung einer HF-Kennungsmarke ausgelesen. HF-Kennungsmarken, insbesondere RFID-Tags, ermöglichen ein sicheres Auslesen des Kennzeichnungsmerkmals, ohne dass eine Erkennungsmarke vorgewiesen werden muss. Insbesondere kann eine derartige Kennungsmarke unsichtbar getragen werden und dennoch zuverlässig ausgelesen werden. Durch eine entsprechende Gestaltung der Auslesecharakteristik kann ferner sichergestellt werden, dass auch bei Vorhandensein mehrerer Kennzeichnungsmarken, nur eine spezifische Marke ausgelesen wird.

Gemäß einer Weiterbildung können eine Vielzahl unterschiedlicher Strichcodes als Kennzeichnungsmerkmal des Probenbehälters verwendet werden, wobei im Kennzeichnungsmerkmal eine eindeutige Kennung codiert hinterlegt ist. Insbesondere kann ein einzelner Strichcode, oder können zwei unterschiedliche Strichcodes vorhanden sein, was eine Kompatibilität mit bestehenden Erfassungsmitteln gewährleistet. Beispielsweise kann der Strichcode aus der Gruppe aus UPC, EAN, EANUCC, CODABAR, CODE 39, CODE 128, Interleaved 2/5, Discrete 2/5, Postnet, BPO, CODE 49, CODE 16K, PDF417, AZTEC, DATAMATRIX und MAXICODE gewählt werden.

Da insbesondere für Probenbehälter im medizinischen Bereich ein zulässiger Verwendungszeitraum nach der Behälterherstellung festgelegt ist, ist es von Vorteil, wenn aus dem Produktionszeitstempel und einem im Auftragsdatensatz hinterlegten Auftrags-datum ein Zeitraum ermittelt wird. Bei Überschreiten eines Verwendungszeitraum-Grenzwertes wird bspw. am Anzeigemittel eine zweite Fehlermeldung ausgegeben. Somit wird mit der Erfassung der Kennung des Probenbehälters ermittelt, ob der Behälter den Anforderungen für die Probenentnahme noch genügt. Beispielsweise kann im Probenbehälter ein bioaktives Material angeordnet sein, welches nur eine maximal zulässige Zeit verwendbar ist.

Zu Zwecken der Qualitätssicherung bzw. zur Nachverfolgung der Abnahmeschritte ist es von Vorteil, wenn bei der Erfassung der eindeutigen Kennung des Probenbehälters ein Erfassungszeitstempel im Auftragsdatensatz hinterlegt wird.

Für manche Analysen kann es erforderlich sein, dass zwischen der Probenentnahme und der Analyse, ein maximaler Zeitraum nicht überschritten wird. Daher wird gemäß einer Weiterbildung, von einem Analysesystem aus dem Erfassungszeitstempel und einem Analysezeitstempel eine Laufzeit ermittelt und bei Überschreiten eines Laufzeit-Maximums eine dritte Fehlermeldung an das Datenverarbeitungssystem übermittelt.

Neben Probenbehältern mit einer bereits vorhandenen eindeutigen Kennung, können aber auch andere Probenbehälter verwendet werden, bspw. Behälter von Fremdanbietern mit einer nicht kompatiblen Kennung. Daher kann gemäß einer Weiterbildung beim Auslesen der mit der Teilmenge relational verknüpften probenbezogenen Datensätze, für einen spezifischen probenbezogenen Datensatz ein Markierungsetikett erzeugt werden. Dieses Etikett kann anschließend am Probenbehälter angeordnet werden bspw. durch Aufkleben, so dass jederzeit auch beliebige andere Behälter mit dem gegenständlichen Verfahren verwendet werden können.

Nach einer Weiterbildung ist vorgesehen, dass die Abgabe einer ersten und/oder zweiten und/oder dritten Fehlermeldung einen Fehlerzustand festlegen, wobei bei einem Fehlerzustand, für den, die Fehlermeldung generierenden probenbezogenen Datensatz des Auftragsdatensatzes, eine Ungültigkeitskennung im Auftragsdatensatz hinterlegt wird. Dies hat den Vorteil, dass im Fehlerfall automatisch Maßnahmen gesetzt werden können um zu verhindern, dass eine fehlerhaft genommene Probe im Analysenablauf verbleibt und so ggf. den gesamten Analyseablauf ungültig machen kann.

Diesbezüglich ist auch eine Weiterbildung von Vorteil, nach der für den, die Fehlermeldung generierenden probenbezogenen Datensatz, ein weiterer Auftragsdatensatz generiert wird. Somit wird im Fehlerfall automatisch eine neue Anforderung die spezifische Probe betreffend generiert, so dass der Analyselauf damit ggf. nicht beeinträchtigt wird. Insbesondere wird somit verhindert, dass eine fehlerhaft genommene Probe erst bei einer nachgelagerten Analyse erkannt wird und dadurch einen bedeutenden Mehraufwand verursachen würde, da der Patient erneut zur Probenentnahme erscheinen müsste.

Um eine Abnahmesequenz der Proben dokumentieren zu können bzw. um für den Abnehmer eine Nachvollziehbarkeit der bereits abgenommenen Proben geben zu können, wird gemäß einer Weiterbildung nach jedem Verfahrensschritt eine Ablauf-Schrittkennzeichnung im Auftragsdatensatz hinterlegt. Somit kann auch im Fall nicht unmittelbar hintereinander genommener Proben stets nachvollzogen werden, welche Proben bereits genommen wurden. Dies kann bspw. dann vorkommen, wenn vor und/oder nach einer Langzeit-Probenentnahme, zusätzliche biologische Proben zu nehmen sind.

Da an einem Datenverarbeitungssystem mehrere Abnehmer arbeiten können, und dadurch mehrere Patienten gleichzeitig betreut werden können, ist gemäß einer Weiterbildung vorgesehen, dass eine dritte Kennung einer weiteren zweiten Person erfasst wird und der mit der zweiten Kennung der zweiten Person verknüpfte Auftragsdatensatz in den Datenspeicher zurückgegeben wird und der mit der dritten Kennung verknüpfte Auftragsdatensatz der weiteren zweiten Person aus dem Datenspeicher geladen und die Teilmenge bzw. das Proben-Kennzeichnungsmerkmal am Anzeigemittel dargestellt wird.

Insbesondere wird mit einer Weiterbildung nach der beim Auslesen des mit der dritten Kennung verknüpften Auftragsdatensatzes die Ablauf-Schrittkennzeichnung ausgelesen erreicht, dass eine Mehrfachbenutzung der Datenverarbeitungseinrichtung, insbesondere der Ein- Ausgabevorrichtung, ermöglicht wird. Dabei ist stets gewährleistet ist, dass jeder Abnehmer unmittelbar seine Arbeitsschritte fortsetzen kann, ohne zuerst aufwändig zum zuletzt durchgeführten Arbeitsschritt navigieren zu müssen.

Eine Weiterbildung besteht ferner darin, dass im analysebezogenen Datensatz für die relational verknüpften probenbezogenen Datensätze eine Reihenfolge hinterlegt ist, wobei die Reihenfolge ausgelesen wird und die Proben-Kennzeichnungsmerkmale der verknüpften probenbezogenen Datensätze, entsprechend der Reihenfolge gereiht, am Anzeigemittel dargestellt werden. Beispielsweise kann es im Zuge einer Probenentnahmesequenz erforderlich sein, dass der Patient etwas zu sich nimmt, wobei anschließend die Reaktion ermittelt werden soll. Daher sind auch die einzelnen Proben in einer spezifischen Reihenfolge abzunehmen, wobei diese Weiterbildung den Vorteil hat, dass dem Abnehmer am Anzeigemittel vorgegeben wird, welche Probe zu entnehmen bzw. welcher Probenbehälter zu verwenden ist. Durch das gegenständliche Verfahren, insbesondere dadurch, dass eine Typinformation aus dem erfassten Kennzeichnungsmerkmal des Probenbehälters ermittelt wird, ist gewährleistet, dass eine fehlerhafte Abnahmereihenfolge der Proben unmittelbar bei der Erfassung der Probenbehälter auffällt und somit direkt Maßnahmen ergriffen werden können, solange der Patient noch vor Ort ist.

Zum besseren Verständnis der Erfindung wird diese anhand der nachfolgenden Figuren näher erläutert.

Es zeigen jeweils in stark schematisch vereinfachter Darstellung:
- Fig. 1 a) und b): eine Darstellung eines Systems, welches die Schritte des gegenständlichen Probenentnahmeverfahrens durchführt;
- Fig. 2: ein vereinfachtes Ablaufdiagramm des gegenständlichen Verfahrens.

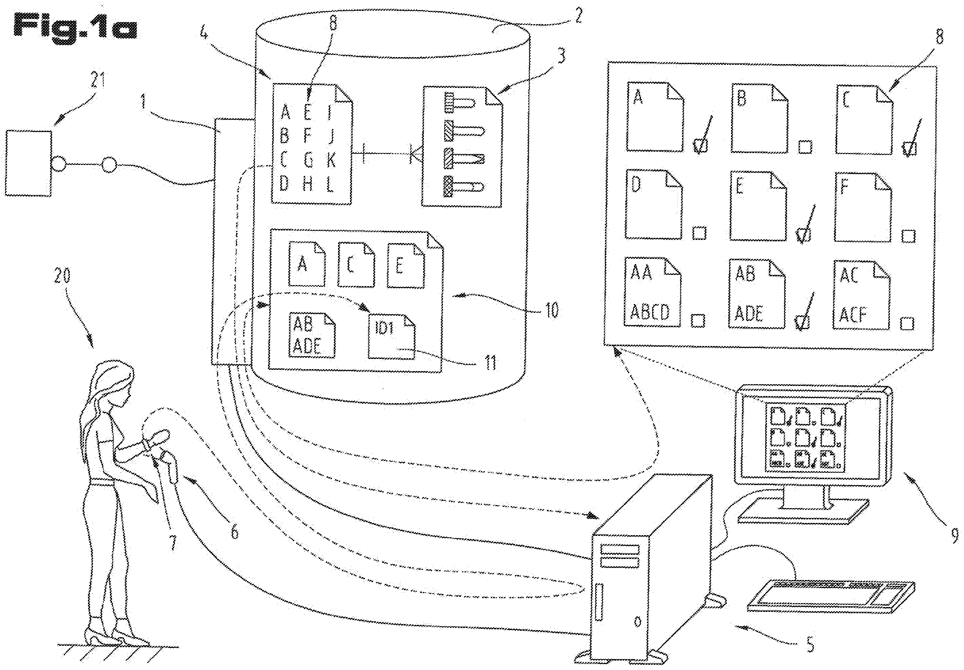

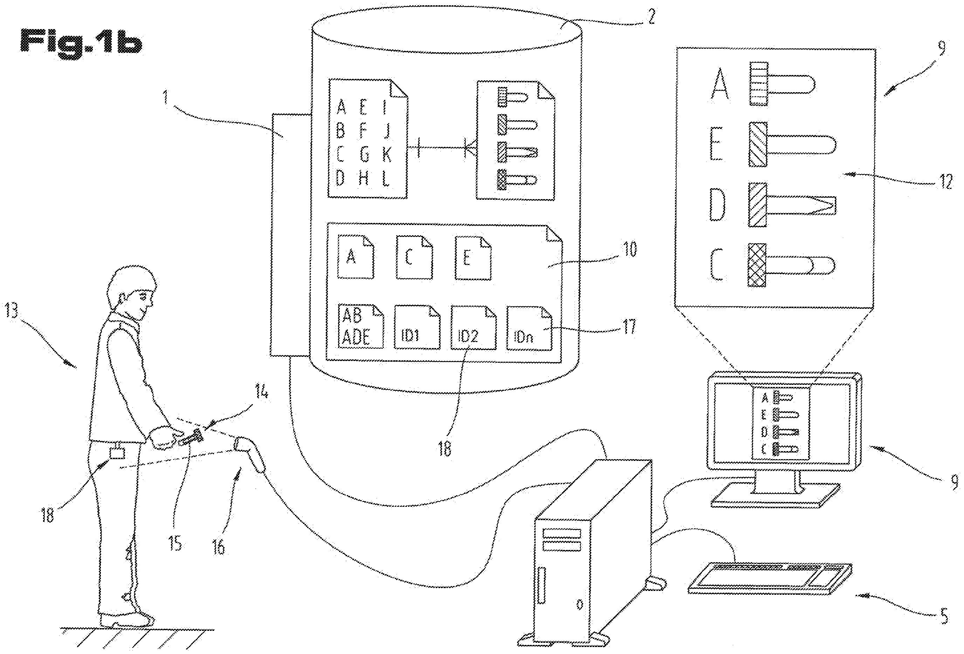

Die Fig. 1a und 1b zeigen schematisch die einzelnen Komponenten einer Vorrichtung zur Durchführung des gegenständlichen Probenentnahmeverfahrens. Das Probenentnahmeverfahren wird dabei auf einem Datenverarbeitungssystem 1 ausgeführt, welches einen Datenspeicher 2 aufweist. Im Datenspeicher 2 sind eine Mehrzahl analysebezogener 4 Datensätze hinterlegt, welche relational mit probenbezogene Datensätzen 3 verknüpft sind. Ferner ist eine Benutzerschnittstelle 5 kommunikativ mit dem Datenverarbeitungssystem 1 verbunden.

Anspruchsgemäß wird als erster Verfahrensschritt ein eindeutiges Kennzeichnungsmerkmal 7 einer ersten Person 20 erfasst. Dies kann beispielsweise dadurch geschehen, dass ein Armband mit einem aufgedruckten bzw. einem integrierten Code ausgelesen wird, wobei eine Ausbildung als Armband mit aufgedrucktem Strichcode, welcher über ein optisches Lesegerät 6 ausgelesen wird, bevorzugt wird. Dieses Lesegerät 6 ist beispielsweise als Laserscanner ausgebildet, welcher weit verbreitet im Einsatz ist und zumeist bei Institutionen, welche Probenentnahmen durchführen, bereits vorhanden ist, sodass keine zusätzliche Neuinvestition erforderlich ist. Das erste Kennzeichnungsmerkmal 7 kann beispielsweise als Armband mit einer Abnahmesicherung ausgebildet sein, sodass ein einmaliges Anordnen an einer Person möglich ist, ein Lösen desselben ist jedoch nur durch Zerstörung des Verschlusses bzw. des Bandes möglich.

Aus dem Datenspeicher werden nun die hinterlegten, analysebezogenen Datensätze 4 ausgelesen und jeweils die im analysebezogenen Datensatz 4 hinterlegte Analysekennung 8 auf einem Anzeigemittel 9 der Ein-Ausgabevorrichtung der Benutzerschnittstelle 5 angezeigt. Für die analysebezogenen Datensätze 4 sind beispielhaft zwei grundlegende Typen dargestellt. Einerseits ist vorgesehen, dass jeder analysebezogene Datensatz mit genau einem probenbezogenem Datensatz 3 korrespondiert, also dass für eine ausgewählte Analyse genau eine Probe zu nehmen ist. Andererseits sind analysebezogene Datensätze vorgesehen, die auf mehrere probenbezogene Datensätze verweisen, sodass bei Auswahl einer derartigen Analyse, mehrere Proben zu nehmen sind. Ein Beispiel dafür ist, wenn für einen Patienten als durchzuführende Analyse eine Analysekennung 8 "großes Blutbild" ausgewählt wird, wobei automatisch durch die relationale Verknüpfung der analysebezogenen Datensätze 4 mit den probenbezogenen Datensätze 3, die entsprechende Auswahl der probenbezogenen Datensätze 3 getroffen wird und somit die erforderliche Anzahl von Probenentnahmen vorgegeben wird.

Ein Bediener wählt nun mit einem Eingabemittel aus der am Anzeigemittel 9 dargestellten Analysekennungen 8 jene aus, welche für den betreffenden Patienten durchgeführt werden müssen. Aus dem erfassten ersten Kennzeichnungsmerkmal 7, insbesondere der damit verknüpften Kennung 11, und der getroffenen Auswahl der Analysekennungen 8, wird vom Datenverarbeitungssystem 1 ein Auftragsdatensatz 10 erstellt und im Datenspeicher 2 hinterlegt. In diesem Auftragsdatensatz 10 sind Referenzkennungen für die ausgewählten und durchzuführenden Analysen (A, C, E, AB), sowie die aus dem ersten Kennzeichnungsmerkmal ausgelesene, eindeutige Kennung 11 der ersten Person hinterlegt.

Fig. 1b zeigt die weiteren Verfahrensschritte. Für den im Datenspeicher 2 hinterlegten Auftragsdatensatz 10 werden die auf die analysebezogenen Datensätze hinterlegten Referenzen analysiert und die mit dieser Teilmenge relational verknüpften, probenbezogenen Datensätze 3 ausgelesen. Die in den probenbezogenen Datensätzen hinterlegte Proben-Kennzeichnungsmerkmale 12 werden dann am Anzeigemittel 9 dargestellt. Bei diesen Proben-Kennzeichnungsmerkmalen handelt es sich bevorzugt jeweils um ein Abbild des betreffenden Probenbehälters. Insbesondere handelt es sich um ein Abbild der Verschlusskappe des Behälters, da die Kappen zur Unterscheidung der Behälter farblich codiert ausgeführt sind und ggf. zusätzliche Farbringe aufweisen. Da aufgrund der durchzuführenden Analysen gegebenenfalls eine Reihenfolge der Probenentnahme einzuhalten ist, werden am Anzeigemittel 9 die Proben-Kennzeichnungsmerkmale 12 in der entsprechenden Verwendungsreihenfolge dargestellt.

Zur Probenentnahme wird von der zweiten Person 13, dem Abnehmer, der erste vorgegebene Probenbehälter genommen und die Probenentnahme durchgeführt und diese Probenentnahme im Auftragsdatensatz dokumentiert. Dazu weist jeder Probenbehälter 14 ein eindeutiges Kennzeichnungsmerkmal 15 auf, welches mittels eines Lesegeräts 16 erfasst und die aus dem ersten Kennzeichnungsmerkmal 15 ausgelesene Kennung 17 im Auftragsdatensatz 10 hinterlegt wird. Zusätzlich wird ein zweites Kennzeichnungsmerkmal 18 der zweiten Person 13 erfasst und die aus dem zweiten Kennzeichnungsmerkmal 18 ausgelesene Kennung 19 ebenfalls wieder im Auftragsdatensatz 10 hinterlegt. Das zweite Kennzeichnungsmerkmal 18 kann beispielsweise durch eine Mitarbeiter-Identifikationskarte gebildet sein, welche die zweite Person 13 als befugten Mitarbeiter der die Probenentnahme durchführenden Institution ausweist. Bevorzugt wird dieses zweite Kennzeichnungsmerkmal 18 ebenfalls wiederum die Kennung in Form eines Strichcodes aufweisen, da damit eine Mehrfachverwendung des Lesegeräts 16 gegeben ist und insbesondere keine zusätzlichen Auslesegeräte angeschafft werden müssen. Das Kennzeichnungsmerkmal 15 des Probenbehälters 14 wird ebenfalls aus Gründen der Kompatibilität bzw. einer Mehrfachverwendung vorhandener Lesegeräte einen Strichcode aufweisen, wobei es insbesondere von Vorteil ist, wenn zwei unterschiedliche Strichcodes aufgebracht sind, da damit einerseits eine Kompatibilität mit bestehenden, am Markt etablierten, Lesegeräten gegeben ist. Ferner kann ein zweiter Strichcode derart ausgebildet sein, zusätzliche Informationen aufnehmen zu können, so dass bspw. eine Rückverfolgung des einzelnen Probenbehälters bis zur Herstellung möglich ist.

Für eine korrekte Durchführung der Analyse ist es nun von Bedeutung, dass einerseits die korrekten Probenbehälter, gegebenenfalls in der entsprechenden Reihenfolge, verwendet werden, und dass insbesondere dem Probenbehälter zugeordnete Verwendungszeitkriterien eingehalten werden. Dazu ist bevorzugt im Kennzeichnungsmerkmal 15 des Probenbehälters 14, bzw. in der hinterlegten Kennung 17, der Typ des Probenbehälters sowie ein Herstellungs- und/oder Verwendungsdatum codiert hinterlegt. Aus dieser Information und aus einer im analysebezogenen Datensatz der Teilmenge hinterlegten Typinformation bzw. aus einer im Auftragsdatensatz 10 hinterlegten bzw. diesem zugeordneten Zeitstempelinformation, kann ein Vergleich durchgeführt werden, ob einerseits der für diese Analyse erforderliche Probenbehälter verwendet wurde bzw. ob bedingt durch Sterilisationsvorgaben, ein festgelegter Verwendungszeitraum eingehalten wurde.

Wird bei dieser Überprüfung ein Fehler festgestellt, wird am Anzeigemittel 9 sofort eine Fehlermeldung ausgegeben, sodass die entsprechende Probenentnahme erneut mit einem korrekten Probenbehälter durchgeführt werden kann. Dies hat insbesondere den Vorteil, dass ein Fehler zu einem Zeitpunkt erkannt wird, wo der Patient noch verfügbar ist und somit die Korrekturmaßnahme kein erneutes Erscheinen des Patienten zur Durchführung der Probenentnahme erforderlich macht.

Die in den Fig. 1a und 1b dargestellten Abläufe können nun kombiniert an einem Ort durchgeführt werden, also dass die erste Person 20 am Durchführungsort der Probenentnahme erscheint und von der zweiten Person 13 sowohl die Erstellung bzw. Auswahl der durchzuführenden Analyse und damit die Bildung des Auftragsdatensatzes durchführt. Ferner kann diese zweite Person 13 anschließend die Probenentnahme und die Zuordnung der Kennungen durchführen.

Mit dem gegenständlichen Probeentnahmeverfahren ist es jedoch auch möglich, dass die Festlegung der Auswahl der durchzuführenden Analysen und damit die Generierung des Auftragsdatensatzes örtlich getrennt von der Durchführung der Probenentnahme erfolgt, beispielsweise kann dies von einem Arzt bei der Visite auf der Station festgelegt werden. Die Probenentnahme und damit die Durchführung der Verfahrensschritte durch die zweite Person 13 finden danach im Labor statt. Da der Auftragsdatensatz und damit alle, die Probenentnahme betreffenden relevanten Datensätze im Datenspeicher 2 eines zentralen Datenverarbeitungssystems 1 hinterlegt sind, können die unterschiedlichsten an der Probenentnahme beteiligten Systeme auf den Auftragsdatensatz 10 zugreifen.

Da ferner ein gegenständliches Probenentnahmeverfahren zumeist in einen übergeordneten Ablauf eingebunden ist und wie es in einem Krankenhaus der Fall ist, von einer Mehrzahl unterschiedlicher Fachabteilungen angefordert werden kann, ist es von Vorteil, wenn das Probenentnahmeverfahren von einem übergeordneten Verwaltungssystem direkt ausgelöst werden kann. Im Gesundheitswesen hat mit HL7 ein Kommunikationsprotokoll weit verbreitet Einsatz gefunden, das es ermöglicht, dass unterschiedliche Systeme über eine Schnittstelle und das gemeinsame Protokoll kommunizieren können. Daher weist das Datenverarbeitungssystem eine Schnittstelle zu einem Prozess-Verwaltungssystem 21 auf, wobei die Kommunikation mit dem Prozess-Verwaltungssystem 21 über diese Schnittstelle bevorzugt mittels HL7 erfolgt. Das Verwaltungssystem 21 kann somit eine Analyseanforderung generieren und diese direkt an das Datenverarbeitungssystem 1 übermitteln, so dass die Verfahrensschritte betreffend die Bildung der Analyse-Teilmenge bereits vom Prozess-Verwaltungssystem durchgeführt wurden. Vom gegenständlichen Verfahren ist es jedenfalls mitumfasst, dass die Verfahrensschritte zur Bildung der Analyse-Teilmenge an der Benutzerschnittstelle 5 des Datenverarbeitungssystem 1 durchgeführt werden, und/oder über eine Schnittstelle vom Prozess-Verwaltungssystem 21 an das Datenverarbeitungssystem 1 übermittelt werden.

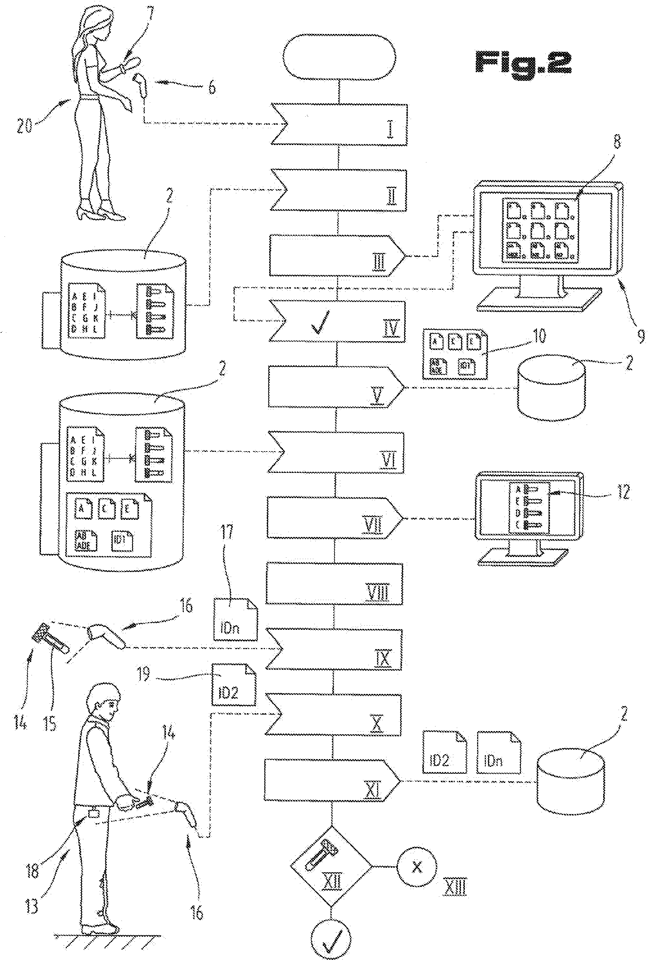

Fig. 2 zeigt eine Darstellung der Verfahrensschritte als schematisiertes Flussdiagramm.

Als erster Verfahrensschritt I nach dem Start wird von einem Lesegerät 6 ein erstes Kennzeichnungsmerkmal 7 einer ersten Person 20 ausgelesen. Danach II werden aus dem Datenspeicher 2 die analysebezogenen Datensätze ausgelesen und im nächsten Schritt III auf einem Anzeigemittel 9 die in den analysebezogenen Datensätzen hinterlegten Analysekennungen 8 dargestellt. Von einem Bediener wird dann mittels eines Eingabegeräts eine Teilmenge der dargestellten Analysekennungen 8 ausgewählt IV, beispielsweise in dem jene Analysekennungen markiert werden, welche korrespondierenden Analysen für den betreffenden Patienten durchzuführen sind. Aus dem ersten Kennzeichnungsmerkmal, insbesondere aus einer daraus ausgelesenen Kennung, und der gewählten Teilmenge der Analysekennungen 8, wird im nächsten Schritt V ein Auftragsdatensatz 10 generiert und im Datenspeicher 2 hinterlegt.

Es wird hier ausdrücklich festgehalten, dass der erste Verfahrensschritt I, also die Erfassung des ersten Kennzeichnungsmerkmals der ersten Person, auch nach der Bildung der Analysenteilmenge IV durchgeführt werden kann.

Danach werden die mit der Teilmenge der Auswahl relational verknüpften probenbezogenen Datensätze aus dem Datenspeicher 2 ausgelesen VI und jeweils ein im probenbezogenen Datensatz hinterlegtes Proben-Kennzeichnungsmerkmal 12, insbesondere ein Abbild, am Anzeigemittel, dargestellt. Der Abnehmer erhält somit die Information, welche Probenbehälter zu verwenden sind, gegebenenfalls wird ihm auch die Probenentnahmereihenfolge vorgegeben.

Nach Durchführung der Probenabnahme VIII wird mittels eines Lesegerätes 16 ein Kennzeichnungsmerkmal 15 des aktuell zur Probenentnahme verwendeten Behälters 14 erfasst und eine in diesem Kennzeichnungsmerkmal 15 hinterlegte Kennung 17 ausgelesen IX. Ebenso wird mit dem Lesegerät 16 ein zweites Kennzeichnungsmerkmal 18 der zweiten Person 13, also des Abnehmers, erfasst und die im Kennzeichnungsmerkmal 18 hinterlegte Kennung 19 ausgelesen X. Die ausgelesene Kennung des Probenbehälters und die ausgelesene Kennung des zweiten Kennzeichnungsmerkmals des Abnehmers werden im Auftragsdatensatz im Datenspeicher 2 hinterlegt XI.

Zu den Verfahrensschritten VIII bis X wird festgehalten, dass die hier dargestellte Reihenfolge einer möglichen Reihenfolge entspricht, dass diese Reihenfolge jedoch zur Erzielung eines effizienteren Ablaufes geändert werden kann. Im Fall einer kombinierten Durchführung des gegenständlichen Probeentnahmeverfahrens an einer Benutzerschnittstelle, kann insbesondere die Erfassung des zweiten Kennzeichnungsmerkmals 18 der zweiten Person 13 (Verfahrensschritt X) auch bereits früher im Ablauf erfolgen, beispielsweise zu Beginn bzw. im Zuge der Erfassung des ersten Kennzeichnungsmerkmals 7 der ersten Person 20.

Aus den im Auftragsdatensatz hinterlegten Kennungen sowie der Auswahl der durchzuführenden Analysen wird nun ein Vergleich durchgeführt XII, um festzustellen, ob der aktuell verwendete Probenbehälter für die durchzuführende Analyse zugelassen ist und ob ferner der für den Probenbehälter zulässige Verwendungszeitraum eingehalten wurde.

Im Fehlerfall XIII wird eine Fehlermeldung ausgegeben und der Abnehmer 13 kann entsprechend reagieren und die Probenentnahme erneut durchführen. Bei einem erfolgreichen Vergleich wird die nächste Probenentnahme durchgeführt (Schritt VIII) oder das Probenentnahmeverfahren korrekt abgeschlossen.

Der Vorteil des gegenständlichen Probenentnahmeverfahrens liegt nun insbesondere darin, dass eine fehlerhafte Probenentnahme aufgrund eines nicht zugelassenen Probenbehälters unmittelbar bei der Probenentnahme erkannt wird, was insbesondere für den Patienten den Vorteil hat, dass dieser nicht erneut zur Probenentnahme erscheinen muss. Ferner ist damit eine lückenlose Erfassung und Nachvollziehbarkeit der Probenentnahme von der Festlegung der Teilmenge der durchzuführenden Analysen bis hin zur Zuordnung des Probenbehälters zur Analyse und insbesondere ferner eine eindeutige Zuordnung der gefüllten Probenbehälter zum Patienten und zum Abnehmer gegeben.

Abschließend sei festgehalten, dass in den unterschiedlich beschriebenen Ausführungsformen gleiche Teile mit gleichen Bezugszeichen bzw. gleichen Bauteilbezeichnungen versehen werden, wobei die in der gesamten Beschreibung enthaltenen Offenbarungen sinngemäß auf gleiche Teile mit gleichen Bezugszeichen bzw. gleichen Bauteilbezeichnungen übertragen werden können. Auch sind die in der Beschreibung gewählten Lageangaben, wie z.B. oben, unten, seitlich usw. auf die unmittelbar beschriebene sowie dargestellte Figur bezogen und sind bei einer Lageänderung sinngemäß auf die neue Lage zu übertragen. Des Weiteren können auch Einzelmerkmale oder Merkmalskombinationen aus den gezeigten und beschriebenen unterschiedlichen Ausführungsbeispielen für sich eigenständige, erfinderische oder erfindungsgemäße Lösungen darstellen.

Sämtliche Angaben zu Wertebereichen in gegenständlicher Beschreibung sind so zu verstehen, dass diese beliebige und alle Teilbereiche daraus mit umfassen, z.B. ist die Angabe 1 bis 10 so zu verstehen, dass sämtliche Teilbereiche, ausgehend von der unteren Grenze 1 und der oberen Grenze 10 mitumfasst sind, d.h. sämtliche Teilbereich beginnen mit einer unteren Grenze von 1 oder größer und enden bei einer oberen Grenze von 10 oder weniger, z.B. 1 bis 1,7, oder 3,2 bis 8,1 oder 5,5 bis 10.

Die Ausführungsbeispiele zeigen mögliche Ausführungsvarianten des Probenentnahmeverfahrens, wobei an dieser Stelle bemerkt sei, dass die Erfindung nicht auf die speziell dargestellten Ausführungsvarianten derselben eingeschränkt ist, sondern vielmehr auch diverse Kombinationen der einzelnen Ausführungsvarianten untereinander möglich sind und diese Variationsmöglichkeit aufgrund der Lehre zum technischen Handeln durch gegenständliche Erfindung im Können des auf diesem technischen Gebiet tätigen Fachmannes liegt. Es sind also auch sämtliche Ausführungsvarianten, die durch Kombinationen einzelner Details der dargestellten und beschriebenen Ausführungsvariante möglich sind und durch die Ansprüche abgedeckt werden, vom Schutzumfang mit umfasst.

Die den eigenständigen erfinderischen Lösungen zugrundeliegende Aufgabe kann der Beschreibung entnommen werden.

Vor allem können die einzelnen in den Figuren gezeigten Ausführungen den Gegenstand von eigenständigen, erfindungsgemäßen Lösungen bilden. Die diesbezüglichen, erfindungsgemäßen Aufgaben und Lösungen sind den Detailbeschreibungen dieser Figuren zu entnehmen.

### Bezugszeichenaufstellung

- 1: Datenverarbeitungssystem
- 2: Datenspeicher
- 3: Probenbezogener Datensatz
- 4: Analysenbezogener Datensatz
- 5: Benutzerschnittstelle

- 6: Lesegerät
- 7: Erstes Kennzeichnungsmerkmal
- 8: Analysekennung
- 9: Anzeigemittel
- 10: Auftragsdatensatz

- 11: Kennung
- 12: Proben-Kennzeichnungsmerkmal
- 13: Zweite Person, Abnehmer
- 14: Probenbehälter
- 15: Kennzeichnungsmerkmal

- 16: Lesegerät
- 17: Kennung
- 18: Zweites Kennzeichnungsmerkmal
- 19: Kennung
- 20: Erste Person, Patient

- 21: Prozess-Verwaltungssystem

## Patentansprüche

1. Probenentnahmeverfahren zur verwechslungssicheren Durchführung einer Probenentnahme ausgeführt auf einem Datenverarbeitungssystem (1) mit einem Datenspeicher (2), und einer mit dem Datenverarbeitungssystem (1) kommunikativ verbundenen Benutzerschnittstelle (5),
wobei
im Datenspeicher (2) eine Mehrzahl probenbezogener (3) und analysebezogener (4) Datensätze hinterlegt sind und wobei die analysebezogenen Datensätze (4) relational mit den probenbezogenen Datensätzen (3) verknüpft sind,
und wobei die Benutzerschnittstelle (5) eine Ein- Ausgabevorrichtung aufweist, welche Ein-Ausgabevorrichtung ein Lesegerät (6) und ein Anzeigemittel (9) aufweist,
mit den Schritten:
• Übermitteln einer Anforderungsanweisung von einem Prozess-Verwaltungssystem (21) an das Datenverarbeitungssystem (1), wobei das Prozess-Verwaltungssystem (21) und das Datenverarbeitungssystem (1) über ein Datenaustauschprotokoll, insbesondere HL7, kommunizieren;
• Erfassen eines ersten Kennzeichnungsmerkmals (7) einer ersten Person (20) durch ein Lesegerät (6), Auslesen der mit dem ersten Kennzeichnungsmerkmal (7) verknüpften Kennung (11) und Übertragen der Kennung (11) an das Datenverarbeitungssystem (1);
• Auslesen der analysebezogenen Datensätze (4) aus dem Datenspeicher (2) und Übertragen zur Benutzerschnittstelle (5) und Darstellen einer Analysekennung (8) am Anzeigemittel (9) der Ein- Ausgabevorrichtung;
• Festlegen einer Teilmenge von Analysen durch Auswählen zumindest einer Analysenkennung (8) mittels der Eingabevorrichtung durch einen Bediener und Übertragen der Teilmenge an das Datenverarbeitungssystem (1);
• Bilden eines Auftragsdatensatzes (10) aus der Kennung (11) und der Teilmenge und Hinterlegen des Auftragsdatensatzes (10) im Datenspeicher (2);
**gekennzeichnet durch** die folgenden Schritte:
• Auslesen des jeweiligen Proben-Kennzeichnungsmerkmals (12), der mit der Teilmenge relational verknüpften probenbezogenen Datensätze (3) und Übertragung zur Benutzerschnittstelle (5) und Darstellung des oder der Proben-Kennzeichnungsmerkmale (12) am Anzeigemittel (9), wobei das Proben-Kennzeichnungsmerkmal (12) eine Abbildung eines Probenbehälters ist;
• Nach Durchführen der Probenentnahme durch eine zweite Person (18), Aktivieren des Erfassungsmittels (6) und Erfassen eines zweiten Kennzeichnungsmerkmals (18) der zweiten Person (13), Auslesen einer Kennung (18) aus dem zweiten Kennzeichnungsmerkmal (18) und Übertragen der Kennung (18) an das Datenverarbeitungssystem (1) und Hinterlegen der Kennung (17) im Auftragsdatensatz (10);
• Erfassen eines Kennzeichnungsmerkmals (15) eines Probenbehälters (14) durch ein Lesegerät (6), Auslesen einer eindeutigen Kennung (17) aus dem Kennzeichnungsmerkmal (15) des Probenbehälters (14), Übertragen der Kennung (17) zum Datenverarbeitungssystem (1) und Hinterlegen im Auftragsdatensatz (10), wobei die eindeutige Kennung (17) des Probenbehälters (14) zumindest durch ein, zum Produktionszeitpunkt des Probenbehälters festgelegtes Merkmal aus der Gruppe Herstellerkennung, Produktionszeitstempel, Verwendungszeitraum, Chargennummer und Artikelnummer ausgebildet ist;
• Auslesen einer Typinformation aus der hinterlegten eindeutigen Kennung (17) des Probenbehälters (14) und Vergleichen mit einer im analysebezogenen Datensatz (4) der Teilmenge im Auftragsdatensatz (10) hinterlegten Typinformation;
• Bei fehlerhafter Übereinstimmung, Ausgeben einer ersten Fehlermeldung am Anzeigemittel.

2. Probenentnahmeverfahren nach Anspruch 1, **dadurch gekennzeichnet, dass** das erste (7) und/oder zweite (13) Kennzeichnungsmerkmal kontaktlos ausgelesen wird.

3. Probenentnahmeverfahren nach Anspruch 2, **dadurch gekennzeichnet, dass** als erstes (7) und/oder zweites (13) Kennzeichnungsmerkmal ein Strichcode einer Kennungsmarke ausgelesen wird.

4. Probenentnahmeverfahren nach Anspruch 2, **dadurch gekennzeichnet, dass** als erstes (7) und/oder zweites (13) Kennzeichnungsmerkmal eine Kennung einer HF-Kennungsmarke ausgelesen wird.

5. Probenentnahmeverfahren nach einem der Ansprüche 1 bis 4, **dadurch gekennzeichnet, dass** als Kennzeichnungsmerkmal (15) des Probenbehälters (14) ein Strichcode aus der Gruppe UPC, EAN, EANUCC, CODABAR, CODE 39, CODE 128, Interleaved 2/5, Discrete 2/5, Postnet, BPO, CODE 49, CODE 16K, PDF417, AZTEC, DATAMATRIX und MAXICODE erfasst wird und daraus eine eindeutige Kennung (17) ausgelesen wird.

6. Probenentnahmeverfahren nach einem der Ansprüche 1 bis 5, **dadurch gekennzeichnet, dass** aus dem Produktionszeitstempel und einem im Auftragsdatensatz (10) hinterlegten Auftragsdatum ein Zeitraum ermittelt wird, wobei bei Überschreiten eines Verwendungszeitraum-Grenzwertes eine zweite Fehlermeldung ausgegeben wird, vorzugsweise am Anzeigemittel.

7. Probenentnahmeverfahren nach einem der Ansprüche 1 bis 6, **dadurch gekennzeichnet, dass** bei der Erfassung des Kennzeichnungsmerkmals (15) des Probenbehälters (14) ein Erfassungszeitstempel im Auftragsdatensatz (10) hinterlegt wird.

8. Probenentnahmeverfahren nach Anspruch 7, **dadurch gekennzeichnet, dass** von einem Analysesystem aus dem Erfassungszeitstempel und einem Analysezeitstempel eine Laufzeit ermittelt wird und bei Überschreiten eines Laufzeit-Maximum eine dritte Fehlermeldung an das Datenverarbeitungssystem (1) übermittelt wird.

9. Probenentnahmeverfahren nach einem der Ansprüche 1 bis 8, **dadurch gekennzeichnet, dass** beim Auslesen der mit der Teilmenge relational verknüpften probenbezogenen Datensätze (3) für einen spezifischen probenbezogenen Datensatz ein Markierungsetikett erzeugt wird.

10. Probenentnahmeverfahren nach einem der Ansprüche 1 bis 9, **dadurch gekennzeichnet, dass** die Abgabe einer ersten und/oder zweiten und/oder dritten Fehlermeldung einen Fehlerzustand festlegen, wobei bei einem Fehlerzustand, für den, die Fehlermeldung generierenden probenbezogenen Datensatz (3) des Auftragsdatensatzes (10), eine Ungültigkeitskennung im Auftragsdatensatz (10) hinterlegt wird.

11. Probenentnahmeverfahren nach Anspruch 10, **dadurch gekennzeichnet, dass** für den, die Fehlermeldung generierenden probenbezogenen Datensatz (3), ein weiterer Auftragsdatensatz (10) generiert wird.

12. Probenentnahmeverfahren nach einem der Ansprüche 1 bis 11, **dadurch gekennzeichnet, dass** nach jedem Verfahrensschritt eine Ablauf-Schrittkennzeichnung im Auftragsdatensatz (10) hinterlegt wird.

13. Probenentnahmeverfahren nach einem der Ansprüche 1 bis 12, **dadurch gekennzeichnet, dass** eine dritte Kennung einer weiteren zweiten Person erfasst wird und der mit der zweiten Kennung (18) der zweiten Person (13) verknüpfte Auftragsdatensatz (10) in den Datenspeicher (2) zurückgegeben wird und der mit der dritten Kennung verknüpfte Auftragsdatensatz der weiteren zweiten Person aus dem Datenspeicher (2) geladen und die Teilmenge bzw. die Proben-Kennzeichnungsmerkmale (12) am Anzeigemittel (9) dargestellt werden.

14. Probenentnahmeverfahren nach Anspruch 12 und 13, **dadurch gekennzeichnet, dass** beim Auslesen des mit der dritten Kennung verknüpften Auftragsdatensatzes die Ablauf-Schrittkennzeichnung ausgelesen wird.

15. Probenentnahmeverfahren nach einem der Ansprüche 1 bis 14, **dadurch gekennzeichnet, dass** im analysebezogenen Datensatz (4) für die relational verknüpften probenbezogenen Datensätze (3) eine Reihenfolge hinterlegt ist, wobei die Reihenfolge ausgelesen wird und die Proben-Kennzeichnungsmerkmale (12) der verknüpften probenbezogenen Datensätze (3), entsprechend der Reihenfolge gereiht, am Anzeigemittel (8) dargestellt werden.

## Claims

1. A sampling method for taking a sample in a manner that reliably avoids mistakes performed on a data processing system (1) with a data memory (2), and a user interface (5) communicatively connected to the data processing system (1),
wherein
in the data memory (2) a plurality of sample-related (3) and analysis-related (4) data records are saved and wherein the analysis-related data records (4) are relationally linked with the sample-related data records (3),
and wherein the user interface (5) has an input/output device, which comprises a reading device (6) and a display means (9),
comprising the steps of:
• transmitting a request instruction from a process management system (21) to the data processing system (1), wherein the process management system (21) and the data processing system (1) communicate via a data exchange protocol, in particular HL7;
• detecting a first identification marker (7) of a first person (20) by means of a reading device (6), reading the identifier (11) associated with the first identification marker (7) and transmitting the identifier (11) to the data processing system (1);
• reading the analysis-related data records (4) from the data memory (2) and transmitting to the user interface (5) and displaying an analysis identifier (8) on a display means (9) of the input/output device;
• determining a subset of analyses by having an operator selecting at least one analysis identifier (8) by means of the input device and transmitting the subset to the data processing system (1);
• forming an order data record (10) from the identifier (11) and a subset and saving the order data record (10) in the data memory (2);
**characterised by** the following steps:
• reading the respective sample identification marker (12) of the sample-related data records (3) relationally linked to the subset and transmitting them to the user interface (5) and displaying the sample identification marker (12) or sample identification markers (12) on the display device (9), wherein the sample identification marker (12) is an image of a sample container;
• following performing the sample taking by a second person (18) activating the detecting device (6) and detecting a second identification marker (18) of the second person (13), reading an identifier (18) from the second identification marker (18) and transmitting the identifier (18) to the data processing system (1) and saving the identifier (17) in the order data record (10);
• detecting an identification marker (15) of a sample container (14) by a reading device (6), reading an unequivocal identifier (17) from the identification marker (15) of the sample container (14), transmitting the identifier (17) to the data processing system (1) and saving in the order data record (10), wherein the unequivocal identifier (17) of the sample container (14) is formed by at least one marker determined at the time of manufacture of the sample container, the marker being formed from the group: manufacturer's identifier, manufacture time stamp, use period, batch number and article number;
• reading a type information from the saved unequivocal identifier (17) of the sample container (14) and comparing with a type information saved in the analysis-related data record (4) of the subset in the order data record (10);
• if there is no match, outputting a first error message on the display means.

2. The sampling method as claimed in claim 1, wherein the first (7) and/or second (13) identification marker is read contactlessly.

3. The sampling method as claimed in claim 2, wherein a barcode of an identifier mark is read as first (7) and/or second (13) identification marker.

4. The sampling method as claimed in claim 2, wherein an identifier of an HF identifier mark is read as a first (7) and/or second (13) identification marker.

5. The sampling method as claimed in one of claims 1 to 4, wherein as identification marker (15) of the sample container (14) a barcode from the group comprising UPC, EAN, EANUCC, CODABAR, CODE 39, CODE 128, Interleaved 2/5, Discrete 2/5, Postnet, BPO, CODE 49, CODE 16K, PDF417, AZTEC, DATAMATRIX and MAXICODE is detected and from the latter an unequivocal identifier (17) is read.

6. The sampling method as claimed in one of claims 1 to 5, wherein from the manufacture time stamp and an order date saved in the order data record (10) a time period is determined, wherein on exceeding a threshold value for the use period a second error message is outputted, preferably on the display means.

7. The sampling method as claimed in one of claims 1 to 6, wherein on the detection of the identification marker (15) of the sample container (14) a detection time stamp is saved in the order data record (10).

8. The sampling method as claimed in claim 7, wherein a run time is determined by an analysis system from the detection time stamp and an analysis time stamp and if a run time maximum is exceeded a third error message is sent to the data processing system (1).

9. The sampling method as claimed in one of claims 1 to 8, wherein on reading the sample-related data records (3) relationally linked to the subset a marking label is produced for a specific sample-related data record.

10. The sampling method as claimed in one of claims 1 to 9, wherein the release of a first and/or second and/or third error message determines an error status, wherein in the case of an error status an invalid marker is saved in the order data record (10) for the sample-related data record (3) of the order data record (10) generating the error message.

11. The sampling method as claimed in claim 10, wherein a further order data record (10) is generated for the sample-related data record (3) generating the error message.

12. The sampling method as claimed in one of claims 1 to 11, wherein after each method step a process step marking is saved in the order data record (10).

13. The sampling method as claimed in one of claims 1 to 12, wherein a third identifier of a further second person is detected and the order data record (10) linked to the second identifier (18) of the second person (13) is returned to the data memory (2), and the order data record of the further second person linked to the third identifier is loaded from the data memory (2) and the subset or the sample identification markers (12) are displayed on the display means (9).

14. The sampling method as claimed in claim 12 and 13, wherein when reading the order data record linked to the third identifier the process step marking is read.

15. The sampling method as claimed in one of claims 1 to 14, wherein a sequence is saved in the analysis-related data record (4) for the relationally linked sample-related data records (3), wherein the sequence is read and the sample identification markers (12) of the linked sample-related data records (3) are shown on the display means (8), arranged in sequence.

## Revendications

1. Procédé de prélèvement d'échantillons pour la réalisation sans risque de confusion d'un prélèvement d'échantillon, exécuté sur un système de traitement de données (1) avec une mémoire de données (2) et une interface utilisateur (5) reliée de manière communicative avec le système de traitement de données (1),
moyennant quoi, dans la mémoire de données (2), sont enregistrés une pluralité d'ensembles de données concernant les échantillons (3) et concernant les analyses (4) et les ensembles de données concernant les analyses (4) sont associés de manière relationnelle avec les ensembles de données concernant les échantillons (3),
et l'interface utilisateur (5) comprend un dispositif d'entrée/sortie, ce dispositif d'entrée/sortie comprenant un appareil de lecture (6) et un moyen d'affichage (9), avec les étapes suivantes :
• transmission d'une demande d'un système de gestion de process (21) au système de traitement de données (1), le système de gestion de process (21) et le système de traitement de données (1) communiquant par l'intermédiaire d'un protocole d'échange de données, plus particulièrement HL7 ;
• mesure d'une première caractéristique d'identification (7) d'une première personne (20) à l'aide d'un appareil de lecture (6), lecture de l'identifiant (11) associé à la première caractéristique d'identification (7) et transmission de l'identifiant (11) au système de traitement de données (1) ;
• lecture des ensembles de données concernant les analyses (4) dans la mémoire de données (2) et transmission à l'interface utilisateur (5) et représentation d'un identifiant d'analyse (8) sur le moyen d'affichage (9) du dispositif d'entrée/sortie ;
• détermination d'une quantité partielle d'analyses par sélection d'au moins un identifiant d'analyse (8) au moyen du dispositif d'entrée par un utilisateur et transmission de la quantité partielle au système de traitement de données (1) ;
• formation d'un ensemble de données de commande (10) à partir de l'identifiant (11) et de la quantité partielle et enregistrement de l'ensemble de données de commande (10) dans la mémoire de données (2) ;
**caractérisé par** les étapes suivantes :
• lecture de la caractéristique d'identification d'échantillon (12) correspondante, des ensembles de données concernant les échantillons (3) associés de manière relationnelle avec la quantité partielle et transmission à l'interface utilisateur (5) et représentation de la ou des caractéristiques d'identification d'échantillons (12) sur le moyen d'affichage (9), la caractéristique d'identification d'échantillon (12) étant une représentation d'un récipient à échantillons ;
• après la réalisation du prélèvement d'échantillon par une deuxième personne (18), activation du moyen de mesure (6) et mesure d'une deuxième caractéristique d'identification (18) de la deuxième personne (13), lecture d'un identifiant (18) à partir de la deuxième caractéristique d'identification (18) et transmission de l'identifiant (18) au système de traitement de données (1) et enregistrement de l'identifiant (17) dans l'ensemble de données de commande (10) ;
• mesure d'une caractéristique d'identification (15) d'un récipient à échantillon (14) à l'aide d'un appareil de lecture (6), lecture d'un identifiant unique (17) à partir de la caractéristique d'identification (15) du récipient à échantillons (14), transmission de l'identifiant (17) au système de traitement de données (1) et enregistrement dans l'ensemble de données de commande (10), l'identifiant unique (17) du récipient à échantillons (14) étant constitué d'au moins une caractéristique déterminée au moment de la production du récipient à échantillons, dans le groupe constitué de : identifiant de fabricant, horodatage de production, période d'utilisation, numéro de lot et numéro d'article ;
• lecture d'une information de type à partir de l'identifiant unique (17) enregistré du récipient à échantillon (14) et comparaison avec une information de type enregistrée dans l'ensemble de données concernant l'analyse (4) de la quantité partielle dans l'ensemble de données de commande (10) ;
• en cas de non-correspondance, sortie d'un message d'erreur au niveau du moyen d'affichage.

2. Procédé de prélèvement d'échantillon selon la revendication 1, **caractérisé en ce que** la première (7) et/ou la deuxième (13) caractéristique d'identification est lue sans contact.

3. Procédé de prélèvement d'échantillon selon la revendication 2, **caractérisé en ce que**, en tant que première (7) et/ou la deuxième (13) caractéristique d'identification, un code-barre d'une marque d'identification est lu.

4. Procédé de prélèvement d'échantillon selon la revendication 2, **caractérisé en ce que**, en tant que première (7) et/ou la deuxième (13) caractéristique d'identification, un identifiant d'une marque d'identification HF est lu.

5. Procédé de prélèvement d'échantillon selon l'une des revendications 1 à 4, **caractérisé en ce que**, en tant que caractéristique d'identification (15) du récipient à échantillons (14), un code-barre sélectionné dans le groupe constitué de : UPC, EAN, EANUCC, CODABAR, CODE 39, CODE 128, Interleaved 2/5, Discrete 2/5, Postnet, BPO, CODE 49, CODE 16K, PDF417, AZTEC, DATAMATRIX et MATRIXCODE est détecté et un identifiant unique (17) est lu à partir de celui-ci.

6. Procédé de prélèvement d'échantillon selon l'une des revendications 1 à 5, **caractérisé en ce que**, à partir de l'horodatage de production et d'une date de commande enregistrée dans l'ensemble de données de commande (10), une période est déterminée, moyennant quoi, lors du dépassement d'une valeur limite de période d'utilisation, un deuxième message d'erreur est généré, de préférence au niveau du moyen d'affichage.

7. Procédé de prélèvement d'échantillon selon l'une des revendications 1 à 6, **caractérisé en ce que**, lors de la détection de la caractéristique d'identification (15) du récipient à échantillon (14), un horodatage de détection est enregistré dans l'ensemble de données de commande (10).

8. Procédé de prélèvement d'échantillon selon la revendication 7, **caractérisé en ce qu'**un temps de marche est déterminé par un système d'analyse à partir de l'horodatage de détection et d'un horodatage d'analyse et, lors du dépassement d'un maximum de temps de marche, un troisième message d'erreur est transmis au système de traitement de données (1).

9. Procédé de prélèvement d'échantillon selon l'une des revendications 1 à 8, **caractérisé en ce que**, lors de la lecture des ensembles de données concernant les échantillons (3) associés de manière relationnelle à la quantité partielle, une étiquette de marquage est générée pour un ensemble de données concernant un échantillon spécifique.

10. Procédé de prélèvement d'échantillon selon l'une des revendications 1 à 9, **caractérisé en ce que** la génération d'un premier et/ou d'un deuxième et/ou d'un troisième message d'erreur détermine un état d'erreur, moyennant quoi, lors d'un état d'erreur pour l'ensemble de données concernant un échantillon (3) générant le message d'erreur de l'ensemble de données de commande (10), un identifiant d'invalidité est enregistré dans l'ensemble de données de commande (10).

11. Procédé de prélèvement d'échantillon selon la revendication 10, **caractérisé en ce que**, pour l'ensemble de données concernant un échantillon (3) générant le message d'erreur, un autre ensemble de données de commande (10) est généré.

12. Procédé de prélèvement d'échantillon selon l'une des revendications 1 à 11, **caractérisé en ce que**, après chaque étape du procédé, un identifiant d'étape de déroulement est enregistré dans l'ensemble de données de commande (10).

13. Procédé de prélèvement d'échantillon selon l'une des revendications 1 à 12, **caractérisé en ce qu'**un troisième identifiant d'une autre deuxième personne est détecté et l'ensemble de données de commande (10) associé avec le deuxième identifiant (18) de la deuxième personne (13) est retourné dans la mémoire de données (2) et l'ensemble de données de commande associé avec le troisième identifiant de l'autre deuxième personne est chargé à partir de la mémoire de données (2) et la quantité partielle ou les caractéristiques d'identification des échantillons (12) sont représentés sur le moyen d'affichage (9).

14. Procédé de prélèvement d'échantillon selon la revendication 12 et 13, **caractérisé en ce que**, lors de la lecture de l'ensemble de données de commande associé avec le troisième identifiant, l'identification d'étape de déroulement est lue.

15. Procédé de prélèvement d'échantillon selon l'une des revendications 1 à 14, **caractérisé en ce que**, dans l'ensemble de données concernant une analyse (4), pour les ensembles de données concernant les échantillons (3) associés de manière relationnelle, une séquence est enregistrée, cette séquence étant lue et les caractéristiques d'identification d'échantillons (12) des ensembles de données concernant les échantillons (3) associés sont représentées sur le moyen d'affichage (8) selon l'ordre de la séquence.
